# EUROPEAN PATENT APPLICATION

(11) **EP 2 679 161 A1**
(43) Date of publication of application: **01.01.2014**
(21) Application number: 12749516.6
(22) Date of filing: 22.02.2012
(51) Int. Cl.: A61B 8/00, G01N 29/00

(54) **ACOUSTO-OPTICAL IMAGE GENERATING DEVICE AND METHOD**

(30) Priority: 22.02.2011 JP 2011035560; 21.12.2011 JP 2011279154
(71) Applicant: FUJIFILM Corporation, Tokyo 106-8620 (JP)
(72) Inventor: HIROTA, Kazuhiro, Ashigarakami-gun Kanagawa 258-8538 (JP); TSUJITA, Kazuhiro, Ashigarakami-gun Kanagawa 258-8538 (JP)
(74) Representative: Klunker . Schmitt-Nilson . Hirsch
(86) International application number: PCT/JP2012/001181
(87) International publication number: WO 2012/114729

(57) **Abstract**

To improve safety with respect to human eyes in a photoacoustic imaging apparatus without providing additional physical means.

[Constitution]

A probe (11) irradiates a light beam guided thereto from a laser unit (13) onto a subject, transmits acoustic waves to the subject, and receives acoustic waves from the subject. The probe (11) transmits and receives acoustic waves prior to generating a photoacoustic image. An imaging means (23) generates a reflected acoustic wave image based on the reception results of ultrasonic waves. A contact state judging means (26) judges whether the probe (11) is in contact with the subject, based on the reflected acoustic wave image. A control means (24) outputs a laser oscillation trigger when the contact state judging means (26) judges that the probe (11) is in contact with the subject, and cause the light beam to be irradiated onto the subject from the probe (11).

## Description

### Technical Field

The present invention is related to a photoacoustic imaging apparatus and a photoacoustic imaging method. More specifically, the present invention is related to a photoacoustic imaging apparatus and a photoacoustic imaging method that irradiate light onto a subject, detect acoustic waves generated within the subject by the irradiation of the light, and generate photoacoustic images.

The ultrasound examination method is known as an image examination method that enables examination of the state of the interior of living organisms in a non invasive manner. Ultrasound examination employs an ultrasound probe capable of transmitting and receiving ultrasonic waves. When the ultrasonic waves are transmitted to a subject (living organism) from the ultrasound probe, the ultrasonic waves propagate through the interior of the living organisms, and are reflected at interfaces among tissue systems. The ultrasound probe receives the reflected ultrasonic waves and images the state of the interior of the subject, by calculating distances based on the amounts of time that the reflected ultrasonic waves return to the ultrasound probe.

Photoacoustic imaging, which images the interiors of living organisms utilizing the photoacoustic effect, is also known. Generally, in photoacoustic imaging, pulsed laser beams are irradiated into living organisms. Biological tissue within the living organisms that absorbs the energy of the pulsed laser beams generates ultrasonic waves (photoacoustic signals) by volume expansion thereof due to heat. An ultrasound probe or the like detects the photoacoustic signals, and constructs photoacoustic images based on the detected signals, to enable to enable visualization of the living organisms based on the photoacoustic signals.

In photoacoustic imaging, it is necessary for comparatively high output laser beams to be irradiated into living organisms. From the viewpoint of safety, it is preferable for the output of the pulsed laser beams to be prevented when a probe is not in contact with the living organisms. In this regard, Patent Document 1 discloses an apparatus provided with a measurement target detecting means that detects measurement targets along an optical path, that irradiates light when the measurement target detecting means detects a measurement target. The properties of the measurement targets can be utilized to detect the measurement targets. For example, the light shielding properties, the reflectivities, unique temperatures, weights, and electrostatic capacities of the measurement targets can be utilized to detect the measurement targets.

### [Background Art Documents]

### [Patent Documents]

[Patent Document 1]
   Japanese Unexamined Patent Publication No. 2009-142320

### Disclosure of the Invention

However, the invention of Patent Document 1 requires additional physical means, such as a combination of a light projector and a light receiver, a temperature sensor, etc., to be provided on a probe in order to detect measurement targets. The addition of such physical means increases cost. In addition, measurement targets cannot be detected when a probe not equipped with these additional physical means is employed.

The present invention has been developed in view of the foregoing circumstances. It is an object of the present invention to provide a photoacoustic imaging apparatus and a photoacoustic imaging method that can improve safety with respect to human eyes without providing additional physical means.

In order to achieve the above object, the present invention provides a photoacoustic imaging apparatus, comprising:
a light source;
a probe that irradiates a subject with a light beam guided from the light source, transmits acoustic waves to the subject, and receives acoustic waves from the subject;
imaging means for generating a photoacoustic image based at least on photoacoustic signals, which are acoustic waves received by the probe in response to the light beam being irradiated onto the subject by the probe;
contact state judging means for judging whether the probe is in contact with the subject prior to photoacoustic image being generated, based on reflected acoustic signals, which are acoustic waves received by the probe in response to the acoustic waves transmitted by the probe; and
control means for causing the light beam to be irradiated onto the subject from the probe when the contact state judging means judges that the probe is in contact with the subject.

The photoacoustic imaging apparatus of the present invention may adopt a configuration, wherein:
the probe includes a plurality of ultrasonic transducers that transmit and receive acoustic waves; and
the contact state judging means judges whether the probe is in contact with the subject, based on reflected acoustic signals received by ultrasonic transducers corresponding to at least a portion of a region to be generated as the photoacoustic image.

A configuration may be adopted, wherein:
the region to be generated as the photoacoustic images is divided into a plurality of blocks; and
the contact state judging means judges whether the probe is in contact with the subject, based on reflected acoustic signals received by ultrasonic transducers corresponding to at least a portion of each of the blocks.

In this case, a configuration may be adopted, wherein:
the region corresponding to the irradiation range of the light beam by the probe is switchable in units of the blocks; and
the control means causes the light beam to be irradiated onto the region corresponding to the blocks when the contact state judging means judges that the probe is in contact with the subject at the region corresponding to the blocks onto which the light beam is to be irradiated.

The contact state judging means may judge whether the probe is in contact with the subject, based on the reflected acoustic signals at a predetermined range in the depth direction of the subject.

The imaging means may further generate a reflected acoustic wave image based on the reflected acoustic signals, and the contact state judging means may judge whether the probe is in contact with the subject employing the generated reflected acoustic wave image. In this case, the contact state judging means may have stored therein a typical reflected acoustic wave image which is generated in a state in which the probe is not in contact with a subject as a reference image, and may judge whether the probe is in contact with the subject based on the degree of similarity between the generated reflected acoustic wave image and the reference image. Alternatively, the contact state judging means may perform feature analysis of the reflected acoustic image, and judge whether the probe is in contact with the subject based on the results of the feature analysis.

As an alternative to employing the reflected acoustic wave image, the contact state judging means may judge whether the probe is in contact with the subject, based on the signal waveform of the reflected acoustic signals. In this case, the contact state judging means may perform feature analysis of the signal waveform of the reflected acoustic signals, and judges whether the probe is in contact with the subject based on the results of the feature analysis. As a further alternative, the contact state judging means may have stored therein a typical signal waveform of received reflected acoustic signals in a state in which the probe is not in contact with a subject as a reference signal waveform, and may judge whether the probe is in contact with the subject based on the degree of similarity between the waveform of the received reflected acoustic signals and the reference signal waveform.

The present invention may adopt a configuration, wherein:
the light source comprises a laser medium, a pumping light source that irradiates a pumping light beam onto the laser medium, a pair of mirrors provided to sandwich the laser medium therebetween to form an optical resonator; and a Q switch provided within the optical resonator.

When the photoacoustic images are generated, the control means may transmit a pumping trigger signal to the light source that causes the pumping light beam to be irradiated onto the laser medium, and may transmit a Q switch trigger signal to the light source that causes the Q switch to be turned ON when the contact state judging means judges that the probe is in contact with the subject.

A configuration may be adopted, wherein:
the light source further comprises a wavelength selecting element provided within the optical resonator, and is capable of outputting a plurality of laser beams having wavelengths different from each other.

A configuration may be adopted, wherein:
the wavelength selecting element comprises a plurality of band pass filters that each transmit light beams having different wavelengths; and
the light source further comprises drive means for driving the wavelength selecting means to sequentially switch the band pass filters which are inserted into the optical path of the optical resonator in a predetermined order.

The wavelength selecting element may be constituted by a rotatable filter body that switches the band pass filter to be inserted into the optical path of the optical resonator by rotational displacement; and the drive means may rotate the rotatable filter body.

A configuration may be adopted, wherein:
the imaging means comprises a two wavelength calculating means that extracts the relationships among signal intensities for each of the laser beams of different wavelengths irradiated onto the subject and received by the probe; and
the photoacoustic images are generated based on the relationships among the signal intensities extracted by the 2 wavelength calculating means.

A configuration may be adopted, wherein:
the imaging means further comprises intensity data extracting means for generating intensity data that represents signal intensities based on photoacoustic signals corresponding to each of the plurality of wavelengths, determines the gradation value of each pixel within the photoacoustic images based on the intensity data, and determines the color that each pixel is displayed in based on the relationship among signal intensities.

A configuration may be adopted, wherein:
the plurality of wavelengths of pulsed laser beams output by the light source includes a first wavelength and a second wavelength; and
the imaging means further comprises complexifying means for generating complex number data, in which one of photoacoustic signals received by the probe when the pulsed laser beam of the first wavelength is irradiated onto the subject and photoacoustic signals received by the probe when the pulsed laser beam of the second wavelength is irradiated onto the subject is designated as an real part and the other is designated as a imaginary part, and reconstructing means for generating reconstructed images from the complex number data by Fourier transform.

The present invention also provides a photoacoustic imaging method, comprising:
a step of transmitting ultrasonic waves onto a subject from a probe that irradiates the subject with a light beam, transmits acoustic waves to the subject, and receives acoustic waves from the subject;
a step of receiving reflected acoustic waves which are reflected by the transmission of the acoustic waves with the probe;
a step of judging whether the probe is in contact with the subject, based on the received reflected acoustic waves;
a step of irradiating the light beam onto the subject when it is judged that the probe is in contact with the subject;
a step of receiving acoustic waves generated within the subject in response to irradiation of the light beam; and
a step of generating a photoacoustic image based on the received acoustic waves generated due to the irradiation of the light beam.

The photoacoustic imaging apparatus and the photoacoustic imaging method of the present invention transmits and receives ultrasonic waves prior to generating a photoacoustic image, and judges whether the probe is in contact with the subject, based on the detection results of the ultrasonic waves. Light is irradiated when the probe is in contact with the subject. Therefore, light being irradiated into space can be prevented, and safety with respect to human eyes can be improved. The present invention judges whether the probe is in contact with the subject based on ultrasonic waves. Therefore, no additional physical means are necessary to judge the contact state of the probe.

### Brief Description of the Drawings

Figure 1 is a block diagram that illustrates a photoacoustic imaging apparatus according to a first embodiment of the present invention.
Figure 2 is a diagram that illustrates an example of an ultrasound image which is generated in a state in which a probe is not in contact with a subject.
Figure 3 is a diagram that illustrates an example of an ultrasound image which is generated in a state in which a probe is in contact with a subject.
Figure 4 is a flow chart that illustrates the steps of an operation to generate a photoacoustic image.
Figure 5 is a diagram that illustrates an example of an image in which a photoacoustic image and an ultrasound image are overlapped.
Figure 6 is a block diagram that illustrates a photoacoustic imaging apparatus according to a second embodiment of the present invention.
Figure 7 is a diagram that illustrates an example of a signal waveform of reflected acoustic signals in a state in which a probe is not in contact with a subject.
Figure 8 is a diagram that illustrates an example of a signal waveform of reflected acoustic signals in a state in which a probe is in contact with a subject.
Figure 9 is a flow chart that illustrates the steps of an operation to generate a photoacoustic image in the second embodiment.
Figure 10 is a block diagram that illustrates a photoacoustic imaging apparatus according to a third embodiment of the present invention.
Figure 11 is a block diagram that illustrates a laser light source unit.
Figure 12 is a block diagram that illustrates a photoacoustic imaging apparatus according to a fourth embodiment of the present invention.
Figure 13 is a block diagram that illustrates a laser light source unit.

### Best Mode for Carrying out the Invention

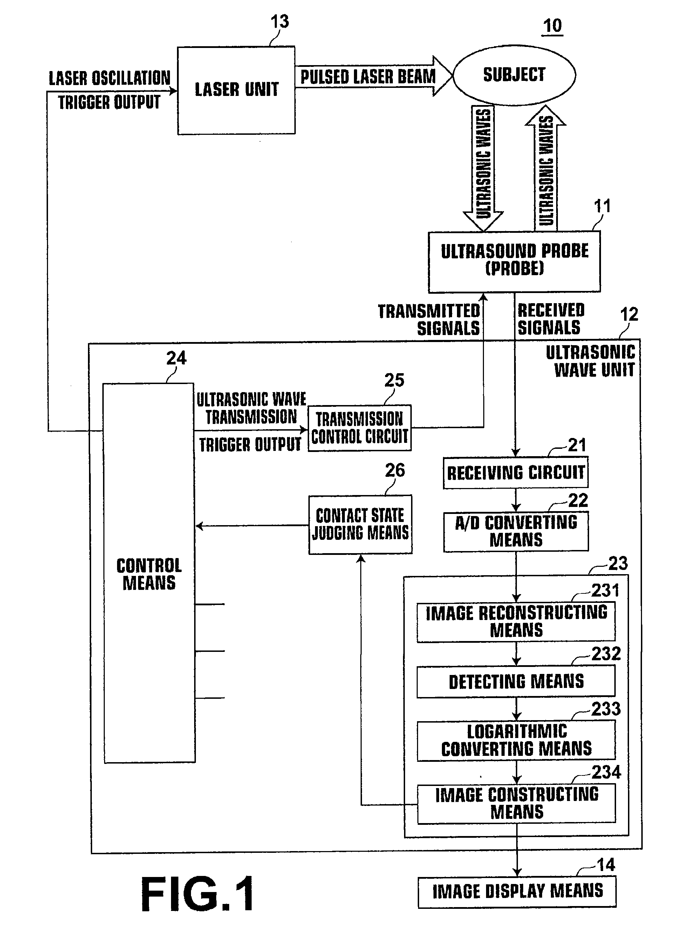

Hereinafter, embodiments of the present invention will be described in detail with reference to the attached drawings. Figure 1 illustrates a photoacoustic imaging apparatus 10 according to a first embodiment of the present invention. The photoacoustic imaging apparatus 10 (photoacoustic image diagnosis apparatus) includes: an ultrasound probe (probe) 11; an ultrasonic wave unit 12; and a light source (laser unit) 13. The photoacoustic image diagnosis apparatus 10 is capable of generating both ultrasound images and photoacoustic images. The laser unit 13 generates a light beam (laser beam) to be irradiated onto a subject when generating a photoacoustic image. The wavelength of the laser beam may be set as appropriate according to observation targets. The laser beam output by the laser unit 13 is guided to the probe 11 by light guiding means, such as an optical fiber.

The probe 11 includes a light emitting section that emits the laser beam guided thereto from the laser unit 13 onto subjects. In addition, the probe 11 outputs (transmits) acoustic waves (ultrasonic waves) to subjects and detects (receives) ultrasonic waves reflected by the subjects. The probe 11 has a plurality of ultrasonic transducers which are arranged one dimensionally, for example. The probe outputs ultrasonic waves from the plurality of ultrasonic transducers when generating ultrasound images, for example, and detects reflected ultrasonic waves (hereinafter, also referred to as "reflected acoustic signals"). The probe 11 detects ultrasonic waves (hereinafter, also referred to as "photoacoustic signals") which are generated by measurement targets within subjects absorbing the laser beam output by the laser unit 13 when generating photoacoustic images.

The ultrasonic wave unit 12 has a receiving circuit 21, an A/D converting means 22, an imaging means 23, a control means 24, a transmission control circuit 25, and a contact state judging means 26. The receiving circuit 21 receives ultrasonic waves (photoacoustic signals or reflected acoustic signals) detected by the plurality of ultrasonic transducers of the probe 11. The A/D converting means 22 converts the ultrasonic wave signals received by the receiving circuit 21 into digital signals. The A/D converting means 22 samples the ultrasonic signals at a predetermined sampling period, for example.

The imaging means 23 generates tomographic images based on the ultrasonic waves sampled by the A/D converting means 22. The imaging means 23 generates photoacoustic images based on photoacoustic signals detected by the probe 11, and generates ultrasound images (reflected acoustic images) based on reflected acoustic signals detected by the probe 11. The imaging means 23 includes an image reconstructing means 231, a detecting means 232, a logarithmic converting means 233, and an image constructing means 234. The functions of each component of the imaging means 23 may be realized by a computer executing processes according to predetermined programs.

The image reconstructing means 231 generates data corresponding to each line of tomographic images, based on the ultrasonic wave signals detected by the plurality of ultrasonic wave transducers of the probe 11. The image reconstructing means 231 adds data from 64 ultrasonic transducers of the probe 11 at delay times corresponding to the positions of the ultrasonic transducers, to generate data corresponding to a single line (delayed addition method), for example. Alternatively, the image reconstructing means 231 may execute image reconstruction by the CBP (Circular Back Projection) method. As further alternatives, the image reconstructing means 231 may execute image reconstruction by the Hough transform method or Fourier transform method.

The detecting means 232 outputs envelope curves for data corresponding to each line output by the image reconstructing means 231. The logarithmic converting means 233 logarithmically converts the envelope curves output by the detecting means 232, to widen the dynamic ranges thereof. The image constructing means 234 generates tomographic images by converting the positions of ultrasonic wave signals (peak positions) along a temporal axis to positions in the depth direction of the tomographic images. An image display means 14 displays the tomographic images generated by the image constructing means 234 to a display monitor or the like.

The control means 24 controls each component of the ultrasonic wave unit 12. The control means 24 outputs an ultrasonic wave transmission trigger signal to the transmission control circuit 25 in the case that the imaging means 23 is to generate an ultrasound image. When the trigger signal is received, the transmission control circuit 25 causes the probe 11 to transmit ultrasonic waves. The control means 24 controls a sampling initiation timing at which the A/D converting means 22 samples reflected acoustic signals such that the sampling initiation timing is synchronized with the ultrasonic wave transmission trigger signal.

Meanwhile, the control means 24 transmits a laser oscillation trigger signal to the laser unit 13 in the case that the imaging means 23 is to generate a photoacoustic image. When the trigger signal is received, the laser unit 13 performs laser oscillation, and outputs a laser beam. The control means 24 controls a sampling initiation timing at which the A/D converting means 22 samples reflected acoustic signals such that the sampling initiation timing is synchronized with the laser oscillation trigger signal.

When a photoacoustic image is to be generated, the control means 24 causes the probe 11 to transmit ultrasonic waves prior to outputting the laser oscillation trigger signal to the laser unit 13. The probe 11 detects the ultrasonic waves which are reflected as reflected acoustic signals. The contact state judging means 26 judges whether the probe 11 is in contact with a subject based on the reflected acoustic signals detected by the probe 11. More specifically, the contact state judging means 26 employs an ultrasound image generated by the imaging means 23 based on the reflected acoustic signals to judge whether the probe 11 is in contact with the subject.

The contact state judging means 26 has stored therein a typical ultrasound image generated in a state in which the probe 11 is not in contact with the subject as a reference image. The contact state judging means 26 compares the ultrasound image generated by the imaging means 23 against the stored reference image, and judges whether the probe 11 is in contact with the subject based on the results of the comparison. The contact state judging means 26 calculates a degree of similarity between the ultrasound image generated by the imaging means 23 and the reference image, for example. The contact state judging means 26 judges that the probe 11 is not in contact with the subject when the degree of similarity between the two ultrasound images is a predetermined threshold value or greater. The contact state judging means 26 judges that the prove 11 is in contact with the subject if the degree of similarity is less than the threshold value.

The control means 24 outputs the laser oscillation trigger signal to the laser unit 13 in the case that the contact state judging means 26 judges that the probe 11 is in contact with the subject, and causes a laser beam to be irradiated onto the subject. In contrast, the control means 24 prevents output of the laser oscillation trigger signal to the laser unit 13 in the case that the contact state judging means 26 judges that the probe 11 is not in contact with the subject, and does not cause a laser beam to be irradiated onto the subject.

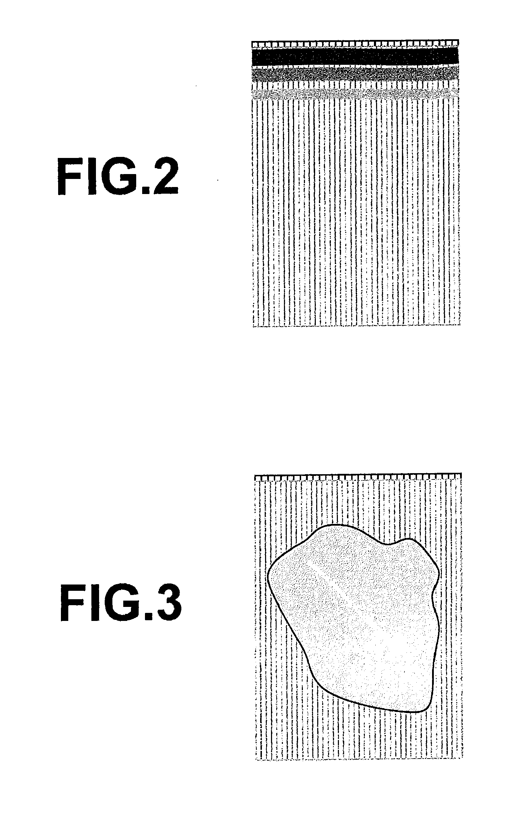

Figure 2 is a diagram that illustrates an example of an ultrasound image which is generated in a state in which the probe 11 is not in contact with a subject. In Figure 2, the horizontal direction of the drawing sheet corresponds to the direction in which the ultrasonic transducers of the probe 11 are one dimensionally arranged, and the vertical direction corresponds to the depth direction. When the probe 11 is in air, that is, when ultrasonic waves are transmitted and received while the probe 11 is not in contact with a subject, and an ultrasound image is generated based on the received ultrasonic waves, the generated ultrasound image will include high brightness echoes at the interface between the probe 11 and air, and multiple echoes thereof.

Figure 3 is a diagram that illustrates an example of an ultrasound image which is generated in a state in which the probe 11 is in contact with a subject. When ultrasonic waves are transmitted and received while the probe 11 is in contact with a subject, the ultrasonic waves transmitted by the probe 11 propagate through the interior of the subject, and reflected waves are generated at the interfaces among tissue systems. Accordingly, when an ultrasound image is generated based on the received ultrasonic waves, the outline of a tissue system will appear in the generated ultrasound image, as illustrated in Figure 3. If the ultrasound image of Figure 2 and the ultrasound image of Figure 3 are compared, it can be understood that generated ultrasound images greatly differ when the probe 11 is in contact with the subject and is not in contact with the subject.

The contact state judging means 26 has stored therein a typical ultrasound image generated in a state in which the probe 11 is not in contact with the subject such as that illustrated in Figure 2 as the reference image. The contact state judging means 26 calculates correlations between the reference image and the ultrasound image generated by the imaging means 23, and judges how similar the images are based on the calculated correlations, for example. The contact state judging means 26 administers a threshold value process on the degree of similarity between the two images, and judges that the generated ultrasound image is an ultrasound image that was generated in a state in which the probe 11 is not in contact with the subject. That is, if the generated ultrasound image is an image such as that illustrated in Figure 2, the contact state judging means 26 judges that the probe 11 was not in contact with the subject when the ultrasound image was generated. In contrast, if the generated ultrasound image is an image such as that illustrated in Figure 3, the contact state judging means 26 judges that the probe 11 was in contact with the subject when the ultrasound image was generated.

A case was described above in which the contact state judging means 26 has the reference image stored therein, and judges whether the probe 11 is in contact with the subject based on the degree of similarity with the reference image. However, the method by which the contact state is judged based on the ultrasound image is not limited to this case. For example, the contact state judging means 26 may perform feature analysis of the ultrasound image generated by the imaging means 23, and may judge whether the probe 11 is in contact with the subject based on the results of the feature analysis. As illustrated in Figure 2, saturated high brightness lines are arrayed parallel to the ultrasonic transducers in an ultrasound image which is generated in a state in which the probe 11 is not in contact with a subject. The contact state judging means 26 may judge that a generated ultrasound image was generated in a state in which the probe 11 is not in contact with a subject in the case that saturated high brightness lines are arrayed parallel to the ultrasonic transducers in the generated ultrasound image.

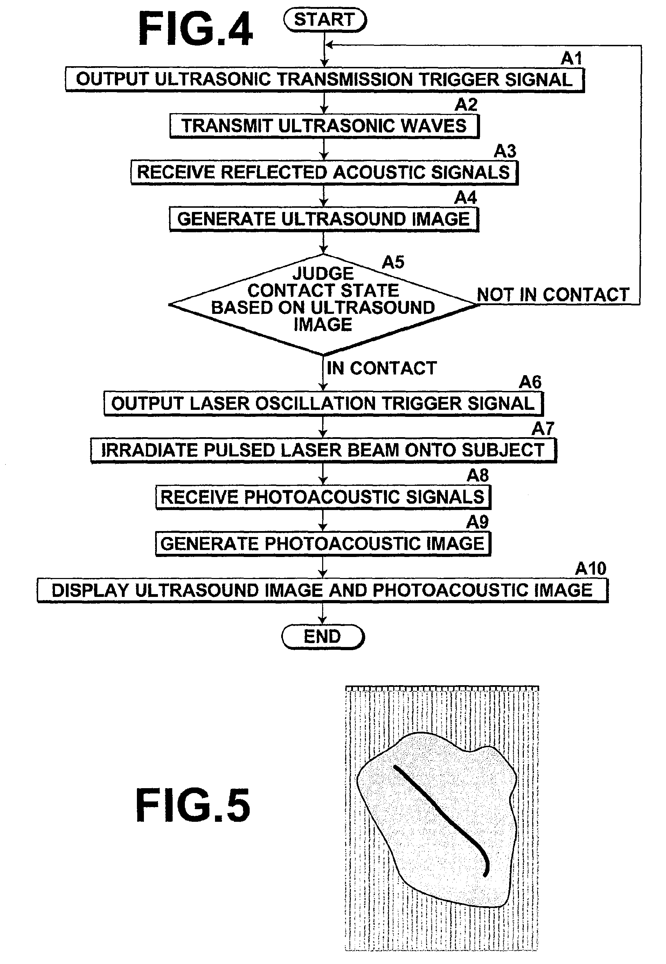

Figure 4 illustrates the steps of an operation for generating a photoacoustic image. The control means 24 outputs an ultrasonic wave transmission trigger signal to the transmission control circuit 25 prior to irradiating a subject with light (step A1). The probe 11 transmits ultrasonic waves into the body of a subject (step A2). The probe 11 receives reflected acoustic signals which are reflected within the body of the subject (Step A3). The imaging means 23 within the ultrasonic wave unit 12 generates an ultrasound image based on the reflected acoustic signals (step A4).

The contact state judging means 26 judges whether the probe 11 is in contact with the subject, based on the ultrasound image which was generated in step A4 (step A5). If it is judged that the probe 11 is not in contact with the subj ect, the operation returns to step A1, and steps A1 through A5 are repeatedly executed until it is judged that the probe 11 is in contact with the subject.

If it is judged that the probe 11 is in contact with the subject at step A5, the control means 24 outputs a laser oscillation trigger signal to the laser unit 13 (step A6). The laser unit 13 outputs a pulsed laser beam after the laser oscillation trigger signal is received. The pulsed laser beam output from the laser unit 13 is irradiated onto the subject by the probe 11 (step A7).

After the laser beam is irradiated, the probe 11 receives photoacoustic signals which are generated within the living body of the subject due to irradiation of the laser beam (step A8) . The imaging means within the ultrasonic wave unit 12 generates a photoacoustic image based on the photoacoustic signals (step A9). The image display means 14 displays the ultrasound image generated at step A4 and the photoacoustic image generated at step A8 on a display screen (step A10) . At step B10, the image display means 14 displays the photoacoustic image and the ultrasound image in an overlapping manner, for example.

Figure 5 is a diagram that illustrates an example of an image in which a photoacoustic image and an ultrasound image are overlapped. In Figure 5, the horizontal direction corresponds to the direction in which the ultrasonic transducers are arranged, and the vertical direction corresponds to the depth direction. As illustrated in Figure 5, the image display means 14 displays the photoacoustic image, in which blood vessels are imaged, overlapped onto the ultrasound image (Figure 3), in which the outline of a tissue system is imaged, for example.

In the present embodiment, whether the probe 11 is in contact with the subject is judged based on the ultrasound image, and the laser oscillation trigger signal is output to the laser unit 13 when the probe 11 is in contact with the subject. Therefore, light being irradiated into space can be prevented, and safety with respect to human eyes can be improved. In addition, the present embodiment judges whether the probe 11 is in contact with the subject based on the ultrasound image. Therefore, no additional physical means such as dedicated sensors for judging a contact state are necessary. In addition, whether the probe 11 is in contact with the subject can be judged even if an existing probe is employed.

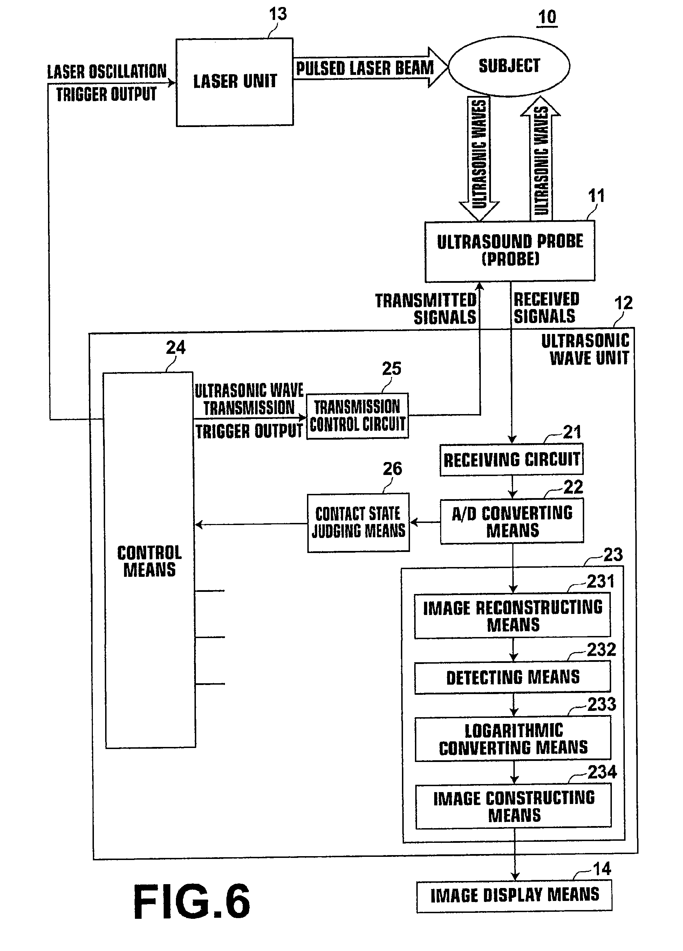

Next, a second embodiment of the present invention will be described. Figure 6 illustrates a photoacoustic imaging apparatus according to the second embodiment of the present invention. The configuration of the photoacoustic imaging apparatus (photoacoustic image diagnosis apparatus) is the same as that of the photoacoustic image diagnosis apparatus of the first embodiment illustrated in Figure 1. In the present embodiment, the contact state judging means 26 judges whether the probe 11 is in contact with the subject based on the signal waveform of reflected acoustic signals instead of ultrasound images. The second embodiment is the same as the

### first embodiment in other points.

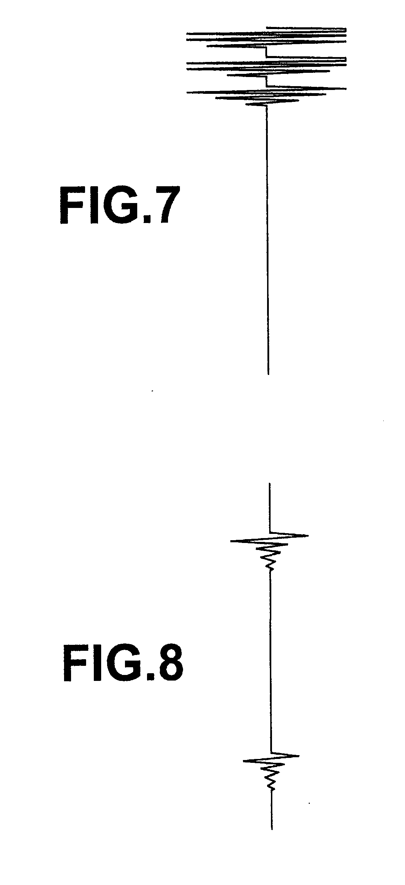

Figure 7 is a diagram that illustrates an example of a signal waveform of reflected acoustic signals in a state in which the probe 11 is not in contact with a subject. In Figure 7, the vertical direction of the drawing sheet corresponds to amounts of time elapsed from initiation of sampling (positions in the depth direction of the subject), and the horizontal direction corresponds to signal levels. When the probe 11 is not in contact with the subject, reflected acoustic signals having a signal waveform in which a plurality of points at which signal levels are saturated appear periodically, as illustrated in Figure 7, are detected. If an ultrasound image is generated based on reflected acoustic signals having such a signal waveform, an ultrasound image such as that illustrated in Figure 2 is obtained.

Figure 8 is a diagram that illustrates an example of a signal waveform of reflected acoustic signals in a state in which the probe 11 is in contact with a subject. In the case that ultrasonic waves are transmitted and received in a state in which the probe 11 is in contact with the subject, the ultrasonic waves transmitted by the probe 11 propagate through the interior of the subject, and reflected waves are generated at the interfaces among tissue systems. In such a case, a signal waveform such as that illustrated in Figure 8 having an amplitude lower than a saturation level is observed. If an ultrasound image is generated based on reflected acoustic signals having such a signal waveform, an ultrasound image such as that illustrated in Figure 3 is obtained. If the signal waveform of reflected acoustic signals illustrated in Figure 7 and the signal waveform of reflected acoustic signals illustrated in Figure 8 are compared, it can be understood that the signal waveforms of reflected acoustic signals greatly differ when the probe 11 is in contact with the subject and is not in contact with the subject.

In the present embodiment, the reflected acoustic signals which are sampled by the A/D converting means 22 are input to the contact state judging means 26. The contact state judging means 26 performs feature analysis of the signal waveform of the reflected acoustic signals, and judges whether the characteristics of a signal waveform of reflected acoustic signals which are observed when the probe 11 is not in contact with a subject are present in the signal waveform of the reflected acoustic signals sampled by the A/D converting means 22. For example, the contact state judging means 26 checks how many locations at which the amplitude of the signal level is greater than or equal to a predetermined level corresponding to a saturation level, and also checks the intervals among locations at which the signal levels are saturated. The contact state judging means 26 may judge that the probe 11 is not in contact with the subject in cases that a plurality of locations at which the signal levels of the reflected acoustic signals are at saturation level are arranged at equidistant intervals, for example. Conversely, the contact state judging means 26 may judge that the probe 11 is in contact with the subject in the case that locations at which signal levels are at saturation level are not arranged at equidistant intervals.

A case has been described above in which the contact state judging means 26 judges whether the probe 11 is in contact with the subject by performing feature analysis of the reflected acoustic signals. However, the method by which the contact state is judged based on the signal waveform of the reflected acoustic signals is not limited to that described above. For example, the contact state judging means 26 may have a typical signal waveform of reflected acoustic signals when the probe 11 is not in contact with a subject such as that illustrated in Figure 7 stored therein as a reference waveform. The contact state judging means 26 may calculate correlations between the reference signal waveform and the signal waveform of the reflected acoustic signals output by the A/D converting means 22, and judge how similar the images are based on the calculated correlations. In this case, the contact state judging means 26 administers a threshold value process on the degree of similarity between the two signal waveforms, judges that the probe 11 is in contact with the subject if the degree of similarity is high, and judges that the probe 11 is in contact with the subject if the degree of similarity is low.

Note signals output by the image reconstructing means 231, the detecting means 232, or the logarithmic converting means 233 may be input to the contact state judging means 26 instead of the reflected acoustic signals sampled by the A/D converting means 22. In these cases as well, it is possible to judge whether the probe 11 is in contact with a subject based on the signal waveform of the reflected acoustic signals prior to generating an ultrasound image.

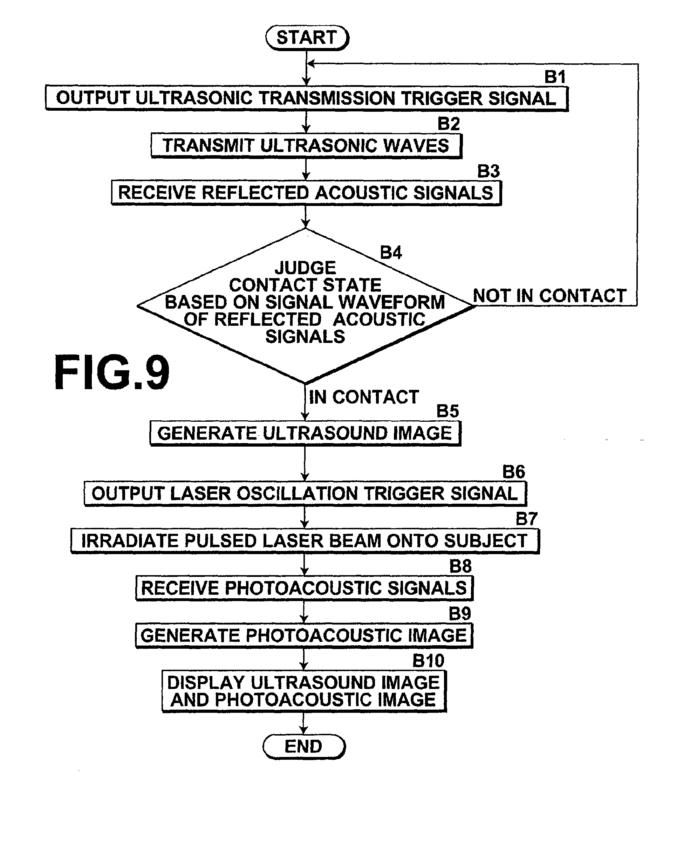

Figure 9 illustrates the steps of an operation for generating a photoacoustic image in the present embodiment. The control means 24 outputs an ultrasonic wave transmission trigger signal to the transmission control circuit 25 prior to irradiating a subject with light (step B1) . The probe 11 transmits ultrasonic waves into the body of a subject (step B2) . The probe 11 receives reflected acoustic signals which are reflected within the body of the subject (Step B3) . The steps up to this point may be the same as steps A1 through A3 of Figure 4.

The contact state judging means 26 judges whether the probe 11 is in contact with the subject, based on the signal waveform of the received reflected acoustic signals (step B4) . The contact state judging means 26 judges whether the prove 11 is in contact with the subject based on the signal waveform of reflected acoustic signals detected by ultrasonic transducers corresponding to at least 1 channel from among the reflected acoustic signals detected by the plurality of ultrasonic transducers of the probe 11. If it is judged that the probe 11 is not in contact with the subject at step B4, the operation returns to step B1, and steps B1 through B4 are repeatedly executed until it is judged that the probe 11 is in contact with the subject. If it is judged that the probe 11 is in contact with the subject at step B4, the imaging means 23 generates an ultrasound image based on the reflected acoustic signals (step B5).

If it is judged that the probe 11 is in contact with the subject at step B4, the control means 24 outputs a laser oscillation trigger signal to the laser unit 13 (step B6). The laser unit 13 outputs a pulsed laser beam after the laser oscillation trigger signal is received. The pulsed laser beam output from the laser unit 13 is irradiated onto the subject by the probe 11 (step B7). After the laser beam is irradiated, the probe 11 receives photoacoustic signals which are generated within the living body of the subject due to irradiation of the laser beam (step B8).

The imaging means within the ultrasonic wave unit 12 generates a photoacoustic image based on the photoacoustic signals (step B9). The image display means 14 displays the ultrasound image generated at step B5 and the photoacoustic image generated at step B8 on a display screen (step B10). At step B10, the image display means 14 displays the photoacoustic image and the ultrasound image in an overlapping manner, for example. Steps B6 through B10 may be the same as steps A6 through A10 of Figure 4. Note that the timing at which the ultrasound image is generated is not limited to immediately after it is judged that the probe 11 is in contact with the subject at step B4, but may be at any desired timing after the reflected acoustic signals are received.

In the present embodiment, whether the probe 11 is in contact with the subj ect is judged based on the signal waveform of reflected acoustic signals instead of an ultrasound image generated from detected reflected acoustic signals. A unique signal waveform is observed when the probe 11 is not in contact with the subject. Therefore, whether the probe 11 is in contact with the subject can be judged without the need for additional physical means in the same manner as in the first embodiment, even if the signal waveform of the reflected acoustic signals is employed prior to generating an ultrasound image. Because the present embodiment can judge the contact state of the probe 11 without generating an ultrasound image, the process for judging the contact state can be simplified compared to that of the first embodiment.

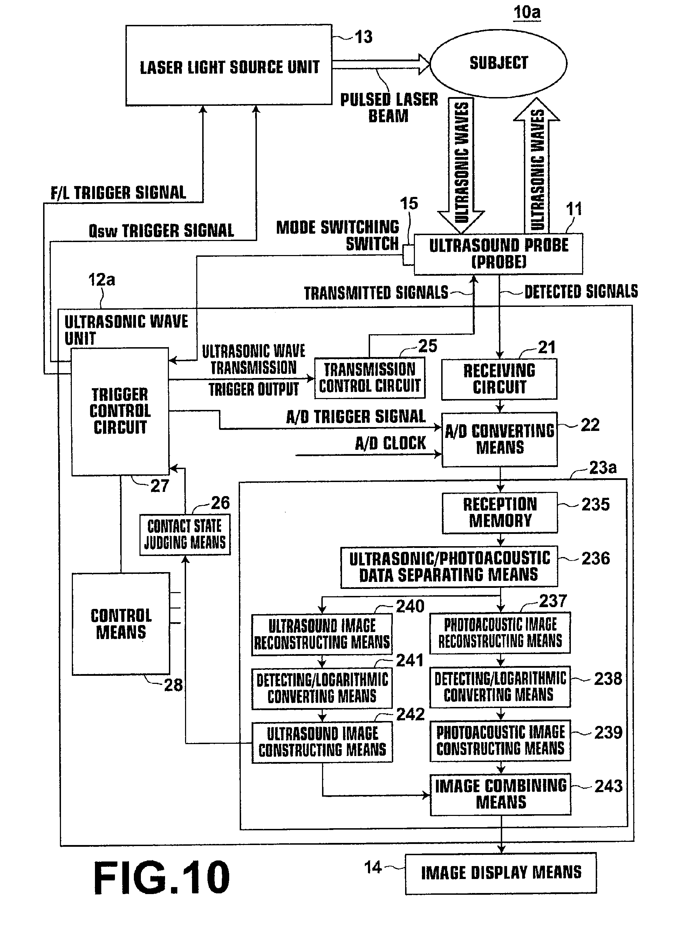

Next, a third embodiment of the present invention will be described. Figure 10 illustrates a photoacoustic imaging apparatus according to the third embodiment of the present invention. The photoacoustic image diagnosis apparatus 10a includes: an ultrasound probe (probe) 11; an ultrasonic wave unit 12a; and a light source (laser unit) 13. The ultrasonic wave unit 12a has: a receiving circuit 21, an A/D converting means 22, an imaging means 23a, a transmission control circuit 25, a contact state judging means 26, a trigger control circuit 27, and a control means 28. The photoacoustic image diagnosis apparatus 10a of the present embodiment is capable of generating both ultrasound images and photoacoustic images in the same manner as the photoacoustic image diagnosis apparatus 10 of the first embodiment illustrated in Figure 1.

The probe 11 includes a light emitting section that emits a laser beam guided thereto from the laser unit 13 onto subjects. In addition, the probe 11 outputs (transmits) ultrasonic waves to subjects and detects (receives) ultrasonic waves reflected by the subjects. The probe 11 has a plurality of ultrasonic transducers which are arranged one dimensionally, for example. The probe 11 has a mode switching switch 15. The probe 11 may be the same as the probe 11 employed by the photoacoustic image diagnosis apparatus 10 of the first embodiment except that the mode switching switch 15 is provided.

The mode switching switch 15 is an alternately operating push switch, for example. The mode switching switch 15 is utilized to switch between operating modes that include photoacoustic image generation, and operating modes that do not include photoacoustic image generation. The operating modes include a mode that generates ultrasound images, a mode that generates photoacoustic images, and a mode that generates ultrasound images and photoacoustic images, for example. An operator, such as a physician, can switch display among ultrasound images, photoacoustic images, and both ultrasound images and photoacoustic images, by pressing the mode switching switch 15.

The receiving circuit 21 receives ultrasonic waves (photoacoustic signals or reflected acoustic signals) detected by the plurality of ultrasonic transducers of the probe 11. The A/D converting means 22 converts the ultrasonic wave signals, that is, the photoacoustic signals and the reflected acoustic signals, received by the receiving circuit 21 into digital signals. The A/D converting means 22 samples the ultrasonic signals at a predetermined sampling period, for example. The imaging means 23 generates tomographic images, that is, photoacoustic images and ultrasound images, based on the ultrasonic waves sampled by the A/D converting means 22.

The imaging means 23a includes: a reception memory 235, a data separating means 236, a photoacoustic image reconstructing means 237, a detecting/logarithmic converting means 238, a photoacoustic image constructing means 239, an ultrasound image reconstructing means 240, a detecting/logarithmic converting means 241, an ultrasound image constructing means 242, and an image combining means 243. The photoacoustic image reconstructing means 237 and the ultrasound image reconstructing means 240 correspond to the image reconstructing means 231 illustrated in Figure 1. The detecting/logarithmic converting means 238 and the detecting/logarithmic converting means 241 correspond to the detecting means 232 and the logarithmic converting means 233 illustrated in Figure 1. The photoacoustic image constructing means 239 and the ultrasound image constructing means 242 correspond to the image constructing means 234 illustrated in Figure 1.

Sampled data of ultrasonic wave signals which are sampled by the A/D converting means 22 are stored in the reception memory 235. That is, photoacoustic data, which are sampled data of photoacoustic signals, and reflected ultrasonic wave data, which are sampled data of reflected acoustic signals, are stored in the reception memory 235. The data separating means 236 separates the photoacoustic data from the reflected ultrasonic wave data within the reception memory 235. The data separating means 236 sends the photoacoustic data to the photoacoustic image reconstructing means 237, and sends the reflected ultrasonic wave data to the ultrasound image reconstructing means 240.

The photoacoustic image reconstructing means 237 reconstructs photoacoustic data. The ultrasound image reconstructing means 240 reconstructs reflected ultrasonic wave data. The reconstruction processes performed by the photoacoustic image reconstructing means 237 and the ultrasound image reconstructing means 240 may be the same as that performed by the image reconstructing means 231 (Figure 1). The detecting/logarithmic converting means 238 detects and administers logarithmic conversion on the photoacoustic data reconstructed by the photoacoustic image reconstructing means 237. The detecting/logarithmic converting means 241 detects and administers logarithmic conversion on the reflected ultrasonic wave data reconstructed by the ultrasound image reconstructing means 240.

The photoacoustic image constructing means 239 generates photoacoustic images based on the detected and logarithmically converted photoacoustic data. The ultrasound image constructing means 242 generates ultrasound images based on the detected and logarithmically converted ultrasonic wave data. The imaging processes performed by the photoacoustic image constructing means 239 and the ultrasound image constructing means 242 may be the same as that performed by the image constructing means 234 (Figure 1). The ultrasound images generated by the ultrasound image constructing means 242 are provided to the contact state judging means 26. The contact state judging means 26 judges whether the probe 11 is in contact with a subject based on the ultrasound images generated by the ultrasound image constructing means 242.

The image combining means 243 combines the photoacoustic images generated by the photoacoustic image constructing means 239 with the ultrasound images generated by the ultrasound image constructing means 242. The image combining means 243 combines the images by overlapping a photoacoustic image on an ultrasound image, for example. At this time, it is preferable for the image combining means 243 to perform alignment such that corresponding points in the photoacoustic image and the ultrasound image are at the same position. The combined images are displayed by an image display means. It is also possible for the image display means 14 to display the photoacoustic image and the ultrasound image arranged next to each other without combining the images, or to switch between display of the photoacoustic image and the ultrasound image.

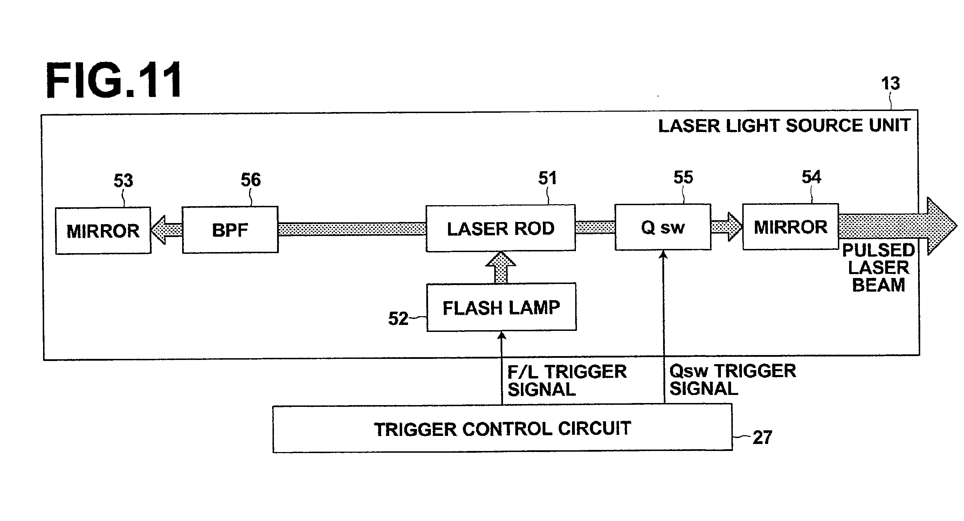

Next, the configuration of the laser unit 13 will be described in detail. Figure 11 illustrates the construction of the laser unit 13. The laser unit 13 has: a laser rod 51, a flash lamp 52, mirrors 53 and 54, a Q switch 55, and a band pass filter 56. The laser rod 51 is a maser medium. An alexandrite crystal, a Cr:LiSAF (Cr:LiSrAlF6), Cr:LiCAF (Cr:LiCaAlF6) crystal, or a Ti:Sapphire crystal may be employed as the laser rod 51. The flash lamp 52 is a pumping light source, and irradiates pumping light onto the laser rod 51. Light sources other than the flash lamp 52, such as semiconductor lasers and solid state lasers, may be employed as the pumping light source.

The mirrors 53 and 54 face each other with the laser rod 51 sandwiched therebetween. The mirrors 53 and 54 constitute an optical resonator. Here, the mirror 54 is an output side mirror. The Q switch 55 is inserted within the resonator. The Q switch 55 changes the insertion loss within the optical resonator from high loss (low Q) to low loss (high Q) at high speed, to obtain a pulsed laser beam. The band pass filter (BPF) 56 selectively transmits light corresponding to wavelengths of the pulsed light beam to be output from the laser unit 13. Elements other than the band pass filter 56, such as a BRF that transmits light of a predetermined wavelength, may be employed. Alternatively, the band pass filter 56 may be omitted.

Returning to Figure 10, the trigger control circuit 27 outputs triggers to switch among operating modes according to an operating state of the mode switching switch 15, triggers to the laser unit 13, triggers to the transmission control circuit 25, and triggers to the A/D converting means 22. The control means 28 is connected to and controls each component within the ultrasonic wave unit 12a. The trigger control circuit 27 and the control means 28 correspond to the control means 24 illustrated in Figure 1.

The trigger control circuit 27 switches operating modes based on signals from the mode switching switch 15. The trigger control circuit 27 switches the operating mode every time that the mode switching switch 15 is operated, for example. The trigger control circuit 27 sets the operating mode to that which does not include photoacoustic image generation, for example, an operating mode that generates only ultrasound images, in an initial state, that is, a state in which the mode switching switch 15 has not been depressed once. If the mode switching switch 15 is depressed while the operating mode is the ultrasound imaging mode, the trigger control circuit 27 switches the operating mode to a photoacoustic imaging mode. If the mode switching switch 15 is depressed again, the trigger control circuit 27 switches the operating mode from the photoacoustic imaging mode to a mode that generates both ultrasound images and photoacoustic images. If the mode switching switch 15 is depressed while in the mode that generates both types of images, the operating mode is returned to the ultrasound imaging mode. Thereafter, the trigger control circuit 27 sequentially switches the operating mode in order from the ultrasound imaging mode, the photoacoustic imaging mode, and the mode that generates both ultrasound images and photoacoustic images.

In addition, the trigger control circuit 27 outputs an ultrasonic wave transmission trigger to the transmission control circuit 25 when transmitting ultrasonic waves to subjects. When the trigger signal is received, the transmission control circuit 25 causes the probe 11 to transmit ultrasonic waves. The trigger control circuit 27 outputs an A/D trigger signal (sampling trigger signal) to the A/D converting means 22 synchronized with the ultrasonic wave transmission trigger signal. When the A/D trigger signal is received, the A/D converting means 22 initiates sampling of reflected acoustic signals.

When the operating mode is that which includes photoacoustic image generation, the trigger control circuit 27 outputs flash lamp trigger signals (pumping trigger signal) that causes the flash lamp 52 (Figure 11) to irradiate a pumping light beam onto the laser rod 51 to the laser unit 13. The trigger control circuit 27 outputs the flash lamp trigger signals at predetermined temporal intervals, for example. The flash lamp 52 irradiates the pumping light beam onto the laser rod in response to the flash lamp trigger signals.

After the pumping light beam is irradiated, if the contact state judging means 26 judges that the probe 11 is in contact with a subject, the trigger control circuit 27 outputs a Q switch trigger signal to the Q switch 55 of the laser unit 13. The Q switch 55 changes the insertion loss within the optical resonator from high loss to low loss at high speed in response to the Q switch trigger signal, to output a pulsed laser beam from the output side mirror 54. The trigger control circuit 27 outputs an A/D trigger signal to the A/D converting means 22 synchronized with the Q switch trigger signal. The A/D converting means 22 initiates sampling of photoacoustic signals when the A/D trigger signal is received.

Meanwhile, the trigger control circuit 27 does not output a Q switch trigger signal while the contact state judging means 11 judges that the probe 11 is not in contact with the subject after output of the flash lamp trigger signal. In this case, the pulsed laser beam is not output, because the Q switch is not turned ON although the laser rod 51 is being pumped. Note that the flash lamp trigger signal and the Q switch trigger signal correspond to the laser oscillation trigger signal of the first embodiment.

In the present embodiment, the laser unit 13 has the Q switch 55 within the optical resonator. The pulses of the laser beam output by the laser unit 13 can be made shorter, by the laser being operated by the Q switch. In addition, in the present embodiment, the trigger control circuit 27 outputs the flash lamp trigger signal regardless of whether the probe 11 is in contact with the subject. The Q switch trigger signal is output if the contact state judging means 26 judges that the probe 11 is in contact with the subject. If the contact state judging means 26 judges that the probe 11 is not in contact with the subject, the laser beam is not output because the Q switch trigger signal is not output although the flash lamp 52 irradiates the pumping light beam onto the laser rod in response to the flash lamp trigger signal. The laser beam is output from the laser unit 13 by the trigger control circuit 27 outputting the Q switch trigger signal when the operating mode includes photoacoustic signal generation and the probe 11 is in contact with the subject.

Here, laser output is not stable immediately after the flash lamp 52 is turned ON. For this reason, it is considered preferable for the flash lamp 52 to periodically continuously emit light. In the present embodiment, control of laser beam output is performed by the Q switch 55, and the laser beam is not output unless the Q switch 55 is turned ON. Therefore, laser output can be prevented while pumping the laser rod 51. For this reason, the flash lamp 52 can be caused to emit light at predetermined intervals regardless of whether the laser beam is output. Use of the Q switch 55 yields the effect of stabilizing laser output in addition to the effect of shortening the pulse of the laser beam, and is greatly advantageous. Further, an effect that the temperature within the optical resonator can be maintained substantially constant can be expected in the case that the flash lamp 52 is caused to continuously emit light at constant intervals. This will result in preventing variations in optical resonating conditions caused by the heat lens effect. The other advantageous effects are the same as those obtained by the first embodiment.

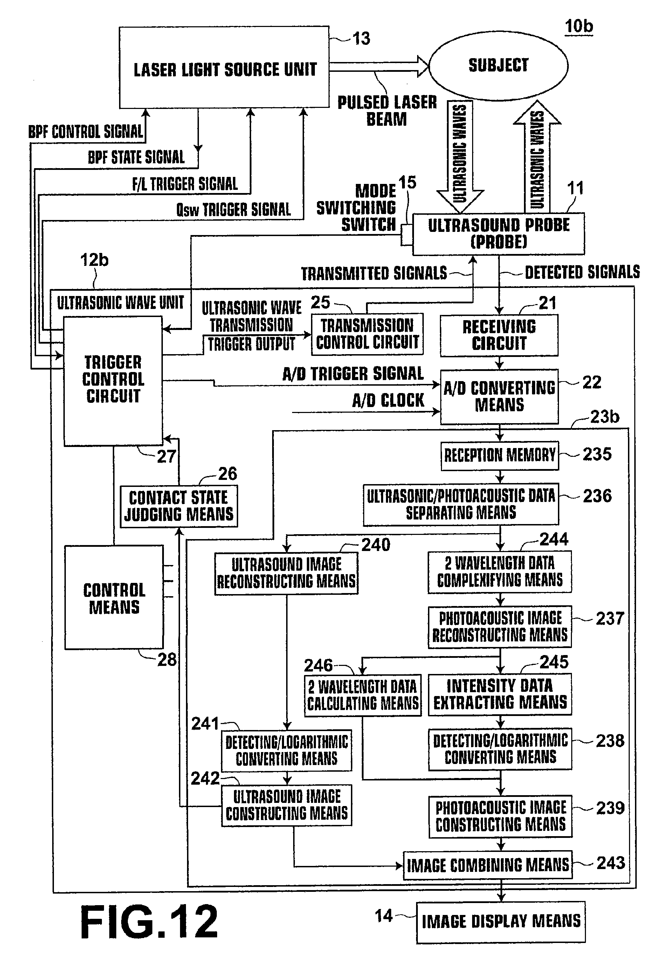

Next, a fourth embodiment of the present invention will be described. Figure 12 illustrates a photoacoustic imaging apparatus 10b according to the fourth embodiment of the present invention. The photoacoustic image diagnosis apparatus 10b of the present embodiment differs from the photoacoustic image diagnosis apparatus 10a of the third embodiment illustrated in Figure 10 in that an imaging means 23b within an ultrasonic wave unit 12b has a 2 wavelength data complexifying means 244, an intensity data extracting means 245, and a two wavelength calculating means 246. In the present embodiment, a laser unit 13b irradiates a plurality of laser beams having different wavelengths onto subjects. The imaging means 23b utilizes the wavelength dependent properties of light absorption characteristics for light absorbers within subjects to generate photoacoustic images in which arteries and veins can be distinguished, for example.

The laser unit 13b of the present embodiment switches output of a plurality of pulsed laser beams having different wavelengths. The pulsed laser beams output from the laser unit 13b are guided to a probe 11 using a light guiding means, such as an optical fiber, and are irradiated onto subjects from the probe 11. A case will be described in which the laser unit 13b sequentially outputs a pulsed laser beam having a first wavelength and a pulsed laser beam having a second wavelength.

A case will be considered in which the first wavelength (central wavelength) is approximately 150nm, and the second wavelength is approximately 800nm. The molecular absorption coefficient of oxidized hemoglobin (hemoglobin bound to oxygen: oxy-Hb), which is contained in human arteries, for a wavelength of 750nm is greater than that for a wavelength of 800nm. Meanwhile, molecular absorption coefficient of deoxidized hemoglobin (hemoglobin not bound to oxygen: deoxy-Hb), which is contained in veins, for a wavelength of 750nm is less than that for a wavelength of 800nm. Photoacoustic signals from arteries and photoacoustic signals from veins can be distinguished by checking the relative intensities of photoacoustic signals obtained for a wavelength of 800nm and photoacoustic signals obtained for a wavelength of 750nm, utilizing these characteristics.

The probe 11 detects ultrasonic wave signals (photoacoustic signals or reflected acoustic signals). A receiving circuit 21 receives the ultrasonic wave signals detected by the probe 11. An A/D converting means 22 samples the ultrasonic wave signals received by the receiving circuit 21. The A/D converting means 22 samples the ultrasonic signals at a predetermined sampling period synchronous with sampling clock signals, for example. The A/D converting means 22 stores reflected ultrasonic wave data, which are the sampled reflected acoustic signals, and photoacoustic data, which are the sampled photoacoustic signals, in a reception memory 235.

With respect to the photoacoustic signals, the A/D converting means 22 stores the sampled photoacoustic data corresponding to each wavelength of the pulsed laser beams output from the laser unit 13b in the reception memory 235. That is, the A/D converting means 22 stores first photoacoustic data, which are the sampled photoacoustic signals that are detected by the probe 11 when the pulsed laser beam having the first wavelength is irradiated onto a subject, and second photoacoustic data, which are the sampled photoacoustic signals that are detected by the probe 11 when the pulsed laser beam having the second wavelength is irradiated onto the subject, in the reception memory 235.

The data separating means 236 separates the ultrasound data, the first photoacoustic data, and the second photoacoustic data, which are stored in the reception memory 235. The data separating means 236 provides the reflected ultrasonic wave data to an ultrasound image reconstructing means 240. The data separating means 236 provides the first and second photoacoustic data to the 2 wavelength data complexifying means 244. The reflected ultrasonic wave data output from the data separating means 236 to the ultrasound image reconstructing means 240 is used to generate an ultrasound image and a contact state judging means 26 judges a contact state of the probe 11 based on the generated ultrasound image in the same manner as in the third embodiment.

The 2 wavelength data complexifying means 244 generates complex number data, in which one of the first photoacoustic data and the second photoacoustic data is designated as a real part, and the other is designated as an imaginary part. Hereinafter, a case will be described in which the 2 wavelength data complexifying means 244 designates the first photoacoustic data as the real part and the second photoacoustic data as the imaginary part.

The complex number data, which the photoacoustic data, are input to a photoacoustic image reconstructing means 237 from the 2 wavelength data complexifying means 244. The photoacoustic image reconstructing means 237 reconstructs the photoacoustic data. The photoacoustic image reconstructing means 237 reconstructs images from the input complex number data by the Fourier transform method (FTA method). Known techniques, such as that disclosed in J. I. Sperl, et al., "Photoacoustic Image Reconstruction - A Quantitative Analysis", SPIE-OSA, Vol. 6631, 663103, 2007, may be applied to image reconstruction by the Fourier transform method. The photoacoustic image reconstructing means 237 inputs data, which have undergone Fourier transform and represent reconstructed images, to the intensity data extracting means 245 and the wavelength data calculating means 246.

The 2 wavelength data calculating means extracts the relative signal intensities between the photoacoustic data corresponding to each wavelength. In the present embodiment, the reconstructed images reconstructed by the photoacoustic image reconstructing means 237 are input to the 2 wavelength data calculating means 246. The 2 wavelength data calculating means 246 extracts phase data that represent which of the real part and the imaginary part is larger and by how much, by comparing the real part and the imaginary part of the input data, which are complex number data. When the complex number data is represented by X+iY, for example, the 2 wavelength data calculating means 246 generates θ=tan⁻¹ (Y/X) as the phase data. Note that θ=90° in the case that X-0. When the first photoacoustic data (X) that constitutes the real part and the second photoacoustic data (Y) that constitutes the imaginary part are equal, the phase data is θ=45°. The phase data becomes closer to θ=0° as the first photoacoustic data is relatively larger, and becomes closer to θ=90° as the second photoacoustic data is relatively larger.

The intensity data extracting means 245 generates intensity data that represent signal intensities, based on the photoacoustic data corresponding to each wavelength. In the present embodiment, the reconstructed images reconstructed by the photoacoustic image reconstructing means 237 are input to the intensity data extracting means 245. The intensity data extracting means 245 generates the intensity data from the input data, which are complex number data. When the complex number data is represented by X+iY, for example, the intensity data extracting means 245 extracts (X²+Y²)^{1/2} as the intensity data. The detecting/logarithmic converting means 238 generates envelope curves of data that represent intensity data extracted by the intensity data extracting means 245, and logarithmically converts the envelope curves to widen the dynamic ranges thereof.

The phase data from the 2 wavelength data calculating means 246 and the intensity data, which have undergone the detection/logarithmic conversion process administered by the detecting/logarithmic converting means 238, are input to the photoacoustic image constructing means 239. The photoacoustic image constructing means 239 generates a photoacoustic image, which is a distribution image of light absorbers, based on the input phase data and intensity data. The photoacoustic image constructing means 239 determines the brightness (gradation value) of each pixel within the distribution image of light absorbers, based on the input intensity data, for example. In addition, the photoacoustic image constructing means 239 determines the color (display color) of each pixel within the distribution image of light absorbers, based on the phase data, for example. The photoacoustic image constructing means 239 employs a color map, in which predetermined colors correspond to a phase range from 0° to 90°, to determine the color of each pixel based on the input phase data for example for example.

Here, the phase range from 0° to 45° is a range in which the first photoacoustic data is greater than the second photoacoustic data. Therefore, the source of the photoacoustic signals may be considered to be arteries, through which blood that mainly contains oxidized hemoglobin having greater absorption with respect to a wavelength of 756nm than a wavelength of 798nm flows. Meanwhile, the phase range from 45° to 90° is a range in which the second photoacoustic data is greater than the first photoacoustic data. Therefore, the source of the photoacoustic signals may be considered to be veins, through which blood that mainly contains deoxidized hemoglobin having lower absorption with respect to a wavelength of 798nm than a wavelength of 756nm flows.

Therefore, a color map, in which a phase of 0° corresponds to red that gradually becomes colorless (white) as the phase approaches 45°, and a phase of 90° corresponds to blue that gradually becomes white as the phase approaches 45°, is employed. In this case, portions corresponding to arteries within the photoacoustic image can be displayed red, and portions corresponding to veins can be displayed blue. A configuration may be adopted, wherein the intensity data are not employed, the gradation values are set to be constant, and portions corresponding to arteries and portions corresponding to veins are merely separated by colors according to the phase data.

The image combining means 243 combines the photoacoustic image generated by the photoacoustic image constructing means and the ultrasound image generated by an ultrasound image constructing means 242. The combined image is displayed by an image display means 14. It is also possible for the image display means 14 to display the photoacoustic image and the ultrasound image arranged next to each other without combining the images, or to switch between display of the photoacoustic image and the ultrasound image.

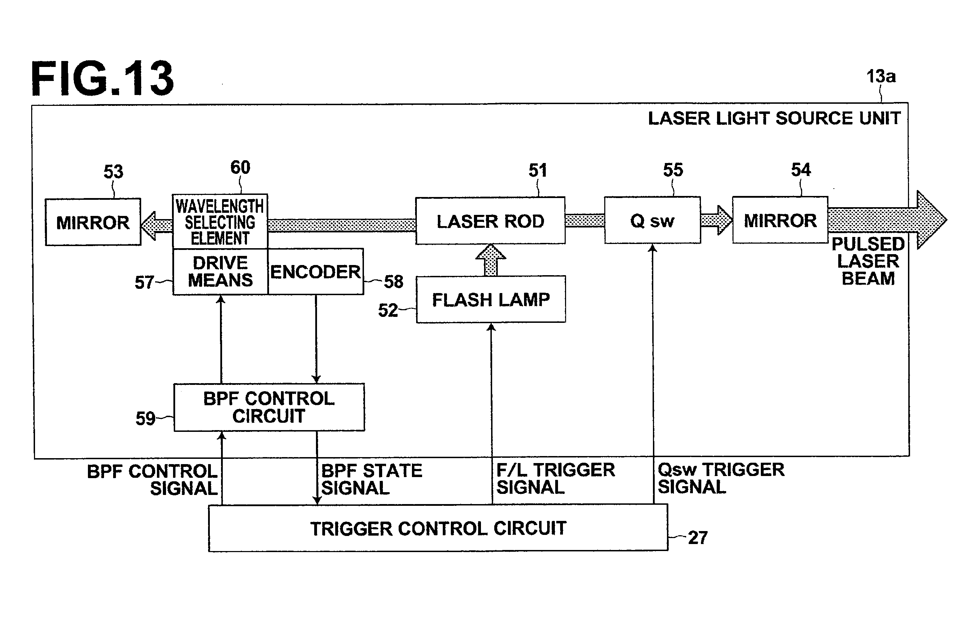

Next, the configuration of the laser unit 13 will be described in detail. Figure 13 illustrates the construction of the laser unit 13b. The laser unit 13 has: a laser rod 51, a flash lamp 52, mirrors 53 and 54, a Q switch 55, a drive means 57, a driving state detecting means 58, a BPF control circuit 59, and a wavelength selecting element 60. The laser rod 51, the flash lamp 52, the mirrors 53 and 54, and the Q switch 55 may be the same as those of the laser unit 13 according to the third embodiment illustrated in Figure 11.

The wavelength selecting element 60 includes a plurality of band pass filters (BPF: Band Pass Filters) that transmit wavelengths different from each other. The wavelength selecting element 60 selectively inserts the plurality of band pass filters into the optical path of the optical resonator. The wavelength selecting element 60 includes a first band pass filter that transmits light having a wavelength of 750nm (central wavelength) and a second band pass filter that transmits light having a wavelength of 800nm (central wavelength), for example. The oscillating wavelength of the laser beam oscillator can be set to 750nm by inserting the first band pass filter into the optical path of the optical oscillator, and the oscillating wavelength of the laser beam oscillator can be set to 800nm by inserting the second band pass filter into the optical path of the optical oscillator.

The drive means 57 drives the wavelength selecting element 60 such that the band pass filters which are inserted into the optical path of the optical resonator are sequentially switched in a predetermined order. A servo motor may be employed as the drive means 57, for example. For example, if the wavelength selecting element 60 is constituted by a rotatable filter body that switches the band pass filter to be inserted into the optical path of the optical resonator by rotational displacement, the drive means 57 rotates the rotatable filter body by rotating a motor output shaft. Half of the rotatable filter body (rotational displacement positions from 0° to 180°, for example) is formed as the first band pass filter that transmits light having a wavelength of 750nm, and the other half of the rotatable filter body (rotational displacement positions from 180° to 360°, for example) is formed as the second band pass filter that transmits light having a wavelength of 800nm, for example. By rotating such a rotatable filter body, the first band pass filter and the second band pass filter can be alternately inserted into the optical path of the optical resonator at a switching speed corresponding to the rotating speed of the rotatable filter body.

The driving state detecting means 58 detects the driving state of the wavelength selecting element 60. The driving state detecting means 58 detects the rotational displacement of the wavelength selecting element 60, which is a rotatable filter body. The driving state detecting means 58 includes a rotary encoder, for example. The rotary encoder detects the rotational displacement of the wavelength selecting element 60, which is a rotatable filter body, with a rotatable plate having a slit mounted on the output shaft of the servo motor, and a transmissive type photo interrupter, and generates BPF state data. The driving state detecting means 58 outputs the BPF state data that represents the rotational displacement position of the rotatable filter body to the BPF control circuit 59.

The BPF control circuit 59 controls voltage which is supplied to the drive means 57 such that the amount of rotational displacement detected by the driving state detecting means 58 within a predetermined type becomes an amount corresponding to a predetermined rotational speed of the rotatable filter body. A trigger control circuit 27 outputs a command that specifies the rotational speed of the rotatable filter body to the BPF control circuit 59, in the form of a BPF control signal. The BPF control circuit 59 monitors the BPF state data and controls the voltage supplied to the servo motor such that the amount of rotational displacement of a rotating shaft of the servo motor detected by the rotary encoder during a predetermined amount of time is maintained at an amount corresponding to the specified rotational speed, for example. The trigger control circuit 27 may be employed instead of the BPF control circuit 59 to monitor the BPF state data and control the drive means 57 such that the wavelength selecting element 60 is driven at a predetermined speed.

Returning to Figure 12, the control means 28 controls each of the components within the ultrasonic wave unit 12b. The trigger control circuit 27 controls the BPF control circuit 59 such that the band pass filters which are inserted into the optical path of the optical resonator within the laser unit 13b are switched at a predetermined switching speed. The trigger control circuit 27 outputs BPF control signals that cause the rotatable filter body that constitutes the wavelength selecting element 60 to rotate continuously in a predetermined direction at a predetermined rotational speed, for example. The rotational speed of the rotatable filter body may be determined based on the number of wavelengths (the number of band pass filters) and the number of pulsed laser beams to be output by the laser unit 13b per unit time.

The trigger control circuit 27 also outputs triggers to switch operating modes according to the operating state of the mode switching switch 15, triggers to the laser unit 13b, triggers to the transmission control circuit 25, and triggers to the A/D converting means 22. The operations for switching operating modes, for outputting triggers to the transmission control circuit 25, and for outputting triggers to the A/D converting means 22 may be the same as those of the trigger control circuit of the third embodiment.

When the operating mode is that which includes photoacoustic image generation, the trigger control circuit 27 outputs flash lamp trigger signals (pumping trigger signal) that causes the flash lamp 52 (Figure 13) to irradiate a pumping light beam onto the laser rod 51 to the laser unit 13. The trigger control circuit 27 outputs the flash lamp trigger signals at predetermined temporal intervals based on BPF state signals. For example, the trigger control circuit 27 outputs a flash lamp trigger signal when the BPF state data represents a position which is the driven position of the wavelength selecting element 60 at which the band pass filter corresponding to the wavelength of a pulsed laser beam to be output minus an amount of displacement that the wavelength selecting element will undergo during an amount of time necessary to pump the laser rod, to cause the pumping light beam to be irradiated onto the laser rod 51. The trigger control circuit 27 outputs the flash lamp trigger signals at periodically at predetermined temporal intervals, for example.

After outputting the flash lamp trigger signal, if the contact state judging means 26 judges that the probe 11 is in contact with a subject, the trigger control circuit 27 outputs a Q switch trigger signal to the Q switch 55 of the laser unit 13b. The trigger control circuit 27 outputs the Q switch trigger signal at a timing at which the band pass filter that transmits a wavelength corresponding to the wavelength of a pulsed laser beam to be output is inserted into the optical path of the optical resonator. For example, in the case that the wavelength selecting element 60 is constituted by a rotatable filter body, the trigger control circuit 27 outputs the Q switch trigger signal when the BPF state data indicates that a band pass filter corresponding to the wavelength of the pulsed laser beam to be output is inserted into the optical path of the optical resonator. The Q switch 55 changes the insertion loss within the optical resonator from high loss to low loss at high speed in response to the Q switch trigger signal, to output a pulsed laser beam from the output side mirror 54. The trigger control circuit 27 outputs an A/D trigger signal to the A/D converting means 22 synchronized with the Q switch trigger signal. The A/D converting means 22 initiates sampling of photoacoustic signals when the A/D trigger signal is received.

Meanwhile, the trigger control circuit 27 does not output a Q switch trigger signal while the contact state judging means 11 judges that the probe 11 is not in contact with the subject after output of the flash lamp trigger signal. In this case, the pulsed laser beam is not output, because the Q switch is not turned ON although the laser rod 51 is being pumped. Note that the flash lamp trigger signal and the Q switch trigger signal correspond to the laser oscillation trigger signal of the first embodiment.

In the present embodiment, the laser unit 13b includes the wavelength selecting element 60, and is capable of irradiating a plurality of laser beams having different wavelengths onto the subject. For example, laser beams having different wavelengths can be continuously switched and output by the laser unit 13b, by continuously driving the wavelength selecting element that includes two band pass filters that transmit different wavelengths, to continuously and selectively insert the two band pass filters into the optical path of the optical resonator. Functional imaging that utilizes the fact that light absorption properties of light absorbers differ according to wavelengths is enabled by employing photoacoustic signals (photoacoustic data) obtained by irradiating pulsed laser beams having different wavelengths.

In the present embodiment, complex number data, in which one of the first photoacoustic data and the second photoacoustic data is designated as a real part and the other is designated as an imaginary part, are generated, and a reconstructed image is generated from the complex number data by the Fourier transform method. In such a case, only a single reconstruction operation is necessary, and reconstruction can be performed more efficiently compared to a case in which the first photoacoustic data and the second photoacoustic data are reconstructed separately.

In the present embodiment as well, the trigger control circuit 27 outputs the flash lamp trigger signal regardless of whether the probe 11 is in contact with the subject. The Q switch trigger signal is output if the contact state judging means 26 judges that the probe 11 is in contact with the subject. The same advantageous effects as those described in connection with the third embodiment are obtained by adopting this configuration. Because the laser beam is not output when the probe 11 is not in contact with the subject, it is possible for the present embodiment to adopt a configuration in which the flash lamp trigger signal is not output when the probe 11 is not in contact with the subject. However, periodically causing the flash lamp 52 to emit light is considered to be advantageous from the viewpoint of laser output stability. Therefore, it is considered that the configuration of the present embodiment is preferable. Driving of the wavelength selecting element 60 may be ceased while the contact state judging means 26 judges that the probe 11 is not in contact with the subject, because the laser beam is not output during this time. In this case, power consumption can be suppressed compared to a case in which the wavelength selecting element is continuously driven.

Note that the embodiments above were described as cases in which the photoacoustic images and the ultrasound images are displayed in an overlapped manner. Alternatively, it is possible for the photoacoustic image diagnosis apparatus to operate in a mode that displays only ultrasound images and in a mode that displays only photoacoustic images. In the mode that displays only ultrasound images, a photoacoustic image may not be generated, an ultrasound image may be generated by executing steps corresponding to steps A1 through A4 of Figure 4, and the ultrasound image may be displayed by the image display means 14, for example. In the mode that displays only photoacoustic images, a photoacoustic image generated at step A9 of Figure 4 may be displayed at step A10, without displaying the ultrasound image generated at step A4 on a display screen, for example.

Particularly in the case that an ultrasound image is not to be displayed on a display screen, the ultrasound image is utilized only to judge the contact state, and therefore it is not necessary for the ultrasound image to be generated for the same range as an imaging range of a photoacoustic image. For example, in step A2 of Figure 4 in the first embodiment, ultrasonic wave transmission may be performed not from all of the ultrasonic transducers of the probe 11, but ultrasonic wave transmission may be performed by ultrasonic transducers corresponding to a portion of an imaging range of a photoacoustic image, and reflected acoustic signals may be detected by the ultrasonic transducers corresponding to the portion at step A3. In this case, the contact state judging means 26 may generate an ultrasound image of the portion for which reflected acoustic signals were obtained in step A4 (generate a partial image based on ultrasonic waves), and judge whether the probe 11 is in contact with a subject based on the partial image.

In greater detail, if there are ultrasonic transducers corresponding to 192 channels, an ultrasound image may be generated for a central portion by transmitting and receiving ultrasonic waves with the central 64 channels, and whether the probe 11 is in contact with a subject may be judged based on the ultrasound image of the central portion. In the second embodiment as well, ultrasonic waves may be transmitted and received not by all of the ultrasonic transducers of the probe 11, but only by ultrasonic transducers corresponding to a portion of an imaging range of a photoacoustic image. In this case, whether the probe 11 is in contact with a subject may be judged based on the ultrasonic wave signal waveform of at least one channel within the range at which ultrasonic waves were transmitted and received, at step B4.

In addition, in the case that the imaging range of photoacoustic images is divided into blocks, the contact state judging means 26 may judge whether the probe is in contact with a subject based on reflected acoustic signals detected by ultrasonic transducers corresponding to at least a portion of each block. For example, if a range of ultrasonic transducers corresponding to 192 channels correspond to an imaging range for a photoacoustic image, the ultrasonic transducers corresponding to 192 channels are divided into three blocks of 64 channels each as a left side block, a center block, and a right side block. An ultrasound image (partial image) of the left side block may be generated based on reflected acoustic signals detected by the ultrasonic transducers of the 64 channels corresponding to the left side block, and whether the left side block is in contact with a subject may be judged based on the partial image. Similarly, partial ultrasound images may be generated corresponding to each of the center block and the right side block, and whether the probe 11 is in contact with the subject may be judged for each block. In the case that the contact state is judged based on the signal waveforms of reflected acoustic signals instead of ultrasound images as well, whether the probe 11 is in contact with a subject may be judged for each block.

For example, in the case that the range onto which the laser beam is irradiated by the probe is switchable in block units, ultrasonic waves may be transmitted and received for at least the blocks that the laser beam is to be irradiated, and the contact state judging means 26 may judge whether the probe 11 is in contact with a subject at these blocks. In this case, the control means 24 may cause the laser beam to be irradiated from the probe 11 onto blocks that the contact state judging means 26 has judged as being in contact with the subject.

Specifically, assume a case in which the probe 11 is capable of switching irradiation of the laser beam onto the left side block, the center block, and the right side block. In the case that the probe is to irradiate the laser beam onto the center block, ultrasonic waves may be transmitted and received at at least the center block, and whether the probe 11 is in contact with a subject at the center block may be judged. When it is judged that the probe 11 is in contact with the subject at the center block, the control means 24 may output a laser oscillation trigger signal to cause the laser beam to be irradiated onto the center block from the probe 11.

In the embodiments described above, ultrasonic waves are transmitted and received to judge whether the probe 11 is in contact with a subject, and an ultrasound image based on reflected acoustic images obtained by the transmission and reception is displayed on the display screen. However, the present invention is not limited to such a configuration. Ultrasonic waves may be transmitted and received separately from the transmission and reception of ultrasonic waves for judging whether the probe 11 is in contact with a subject, and a separate ultrasound image may be generated based on reflected acoustic signals obtained by the separate transmission and reception of ultrasonic waves. For example, after a photoacoustic image is generated in step A9 of Figure 4, ultrasonic waves may be transmitted from the probe 11, reflected acoustic signals may be detected by the probe 11, and the imaging means 23 may generate a separate ultrasound image based on the reflected acoustic signals. In this case, the ultrasound image which was generated separately after the photoacoustic image was generated may be displayed in step A10 instead of the ultrasound image generated in step A4.

The contact state judging means 26 may judge whether the probe 11 is in contact with a subject based on reflected acoustic signals from a predetermined range in the depth direction of the subject. For example, in the first embodiment, the imaging means 23 may generate an ultrasound image within a range from 5mm to 10mm from the surface of the subject, and the contact state judging means 26 may judge the contact state of the probe 11 based on the ultrasound image generated for this range. In addition, in the second embodiment, the A/D converting means 22 may sample reflected acoustic signals for a temporal region corresponding to depths within a range from 5mm to 10mm from the surface of the subject, and the contact state judging means 26 may judge the contact state of the probe 11 based on the signal waveform of the sampled reflected acoustic signals.

The fourth embodiment was described as a case in which the first photoacoustic data and the second photoacoustic data are complexified. Alternatively, the first photoacoustic and the second photoacoustic data may be reconstructed separately without administering the complexifying operation. In addition, the reconstruction method is not limited to the Fourier transform method. Further, the fourth embodiment calculates the ratio between the first photoacoustic data and the second photoacoustic data by employing the phase data obtained by the complexifying operation. However, the same effects can be obtained by calculating the ratio using the intensity data of the first and second photoacoustic data. In addition, the intensity data may be generated based on signal intensities within a first reconstructed image and signal intensities within a second reconstructed image.

The number of pulsed laser beams having different wavelengths which are irradiated onto a subject when generating photoacoustic images is not limited to two. Three or more pulsed laser beams may be irradiated onto the subject, and photoacoustic images may be generated based on photoacoustic data corresponding to each wavelength. In this case, the 2 wavelength data calculating means 246 may generate the relationships among signal intensities of photoacoustic data corresponding to each wavelength as phase data. In addition, the intensity data extracting means 245 may generate a sum of signal intensities of photoacoustic data corresponding to each wavelength as intensity data.

The fourth embodiment was described mainly as a case in which the wavelength selecting element 60 is constituted by a rotatable filter body having two band pass filter regions. However, it is only necessary for the wavelength selecting element 60 to be that which can change the wavelength of light that oscillates within the optical resonator, and is not limited to a rotatable filter body. For example, the wavelength selecting element may be constituted by a rotatable body having a plurality of band pass filters provided on the circumference thereof. It is not necessary for the wavelength selecting element 60 to be a rotatable body. For example, a plurality of band pass filters may be arranged in a row. In this case, the wavelength selecting element 60 may be driven such that the plurality of band pass filters are cyclically inserted into the optical path of the optical resonator, or the wavelength selecting element 60 may be reciprocally driven such that the plurality of band pass filters arranged in a row traverse the optical path of the optical resonator. As a further alternative, a wavelength selecting element such as a birefringent filter may be employed instead of the band pass filters.

Preferred embodiments of the present invention have been described above. However, the photoacoustic imaging apparatus and the photoacoustic imaging method are not limited to the above embodiments. Various changes and modifications to the configurations of the above embodiments are included in the scope of the present invention.

## Claims

1. A photoacoustic imaging apparatus, comprising:
a light source;
a probe that irradiates a subject with a light beam guided from the light source, transmits acoustic waves to the subject, and receives acoustic waves from the subject;
imaging means for generating a photoacoustic image based at least on photoacoustic signals, which are acoustic waves received by the probe in response to the light beam being irradiated onto the subject by the probe;
contact state judging means for judging whether the probe is in contact with the subject prior to generating the photoacoustic image, based on reflected acoustic signals, which are acoustic waves received by the probe in response to the acoustic waves transmitted by the probe; and
control means for causing the light beam to be irradiated onto the subject from the probe when the contact state judging means judges that the probe is in contact with the subject.

2. A photoacoustic imaging apparatus as defined in Claim 1, wherein:
the probe includes a plurality of ultrasonic transducers that transmit and receive acoustic waves; and
the contact state judging means judges whether the probe is in contact with the subject, based on reflected acoustic signals received by ultrasonic transducers corresponding to at least a portion of a region to be generated as the photoacoustic image.

3. A photoacoustic imaging apparatus as defined in Claim 2, wherein:
the region to be generated as the photoacoustic image is divided into a plurality of blocks; and
the contact state judging means judges whether the probe is in contact with the subject, based on reflected acoustic signals received by ultrasonic transducers corresponding to at least a portion of each of the blocks.

4. Aphotoacoustic imaging apparatus as defined in Claim 3, wherein:
the region corresponding to the irradiation range of the light beam by the probe is switchable in units of the blocks; and
the control means causes the light beam to be irradiated onto the region corresponding to the blocks when the contact state judging means judges that the probe is in contact with the subject at the region corresponding to the blocks onto which the light beam is to be irradiated.

5. A photoacoustic imaging apparatus as defined in any one of Claims 1 through 4, wherein:
the contact state judging means judges whether the probe is in contact with the subject, based on the reflected acoustic signals at a predetermined range in the depth direction of the subject.

6. A photoacoustic imaging apparatus as defined in any one of Claims 1 through 5, wherein:
the imaging means further generates a reflected acoustic wave image based on the reflected acoustic signals; and
the contact state judging means judges whether the probe is in contact with the subject employing the generated reflected acoustic wave image.

7. A photoacoustic imaging apparatus as defined in Claim 6, wherein:
the contact state judging means has stored therein a typical reflected acoustic wave image which is generated in a state in which the probe is not in contact with a subject as a reference image, and judges whether the probe is in contact with the subject based on the degree of similarity between the generated reflected acoustic wave image and the reference image.

8. A photoacoustic imaging apparatus as defined in any one of Claims 1 through 5, wherein:
the contact state judging means judges whether the probe is in contact with the subject, based on the signal waveform of the reflected acoustic signals.

9. Aphotoacoustic imaging apparatus as defined in Claim 8, wherein:
the contact state judging means performs feature analysis of the signal waveform of the reflected acoustic signals, and judges whether the probe is in contact with the subject based on the results of the feature analysis.

10. A photoacoustic imaging apparatus as defined in any one of Claims 1 through 9, wherein:
the light source comprises a laser medium, a pumping light source that irradiates a pumping light beam onto the laser medium, a pair of mirrors provided to sandwich the laser medium therebetween to form an optical resonator; and a Q switch provided within the optical resonator.

11. A photoacoustic imaging apparatus as defined in Claim 10, wherein:
when the photoacoustic image is generated, the control means transmits a pumping trigger signal to the light source that causes the pumping light beam to be irradiated onto the laser medium, and transmits a Q switch trigger signal to the light source that causes the Q switch to be turned ON when the contact state judging means judges that the probe is in contact with the subject.

12. A photoacoustic imaging apparatus as defined in either one of Claim 10 and Claim 11, wherein:
the light source further comprises a wavelength selecting element provided within the optical resonator, and is capable of outputting a plurality of laser beams having wavelengths different from each other.

13. A photoacoustic imaging apparatus as defined in Claim 12, wherein:
the wavelength selecting element comprises a plurality of band pass filters that each transmit light beams having different wavelengths; and
the light source further comprises drive means for driving the wavelength selecting means to sequentially switch the band pass filters which are inserted into the optical path of the optical resonator in a predetermined order.

14. A photoacoustic imaging apparatus as defined in Claim 13, wherein:
the wavelength selecting element is constituted by a rotatable filter body that switches the band pass filter to be inserted into the optical path of the optical resonator by rotational displacement; and
the drive means rotates the rotatable filter body.

15. A photoacoustic imaging method, comprising:
a step of transmitting acoustic waves onto a subject from a probe that irradiates the subject with a light beam, transmits acoustic waves to the subj ect, and receives acoustic waves from the subject;
a step of receiving reflected acoustic waves which are reflected by the transmission of the acoustic waves with the probe;
a step of judging whether the probe is in contact with the subject, based on the received reflected acoustic waves;
a step of irradiating the light beam onto the subject when it is judged that the probe is in contact with the subject;
a step of receiving acoustic waves generated within the subject in response to irradiation of the light beam; and
a step of generating a photoacoustic image based on the received acoustic waves generated due to the irradiation of the light beam.
